(19) Europäisches Patentamt
European Patent Office
Office européen des brevets

(11) **EP 3 308 714 B1**

(12) **EUROPEAN PATENT SPECIFICATION**

(45) Date of publication and mention
of the grant of the patent:
**11.12.2019 Bulletin 2019/50**

(51) Int Cl.:
*A61B 8/08* *(2006.01)*    *A61B 8/14* *(2006.01)*

(21) Application number: **17195843.2**

(22) Date of filing: **11.10.2017**

(54) **ULTRASONIC IMAGING APPARATUS AND METHOD FOR MEASURING HARDNESS**

ULTRASCHALLABBILDUNGSVORRICHTUNG UND VERFAHREN ZUR MESSUNG VON HÄRTE

APPAREIL D'IMAGERIE ULTRASONORE ET PROCÉDÉ DE MESURE DE LA DURETÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.10.2016 JP 2016200767**

(43) Date of publication of application:
**18.04.2018 Bulletin 2018/16**

(73) Proprietor: **Hitachi, Ltd.**
**Tokyo 100-8280 (JP)**

(72) Inventors:
• **TANAKA, Tomohiko**
  **Tokyo, 100-8280 (JP)**
• **MARUOKA, Takashi**
  **Tokyo, 100-8280 (JP)**

(74) Representative: **MERH-IP Matias Erny Reichl Hoffmann**
**Patentanwälte PartG mbB**
**Paul-Heyse-Strasse 29**
**80336 München (DE)**

(56) References cited:
**EP-A1- 2 537 469**    **JP-A- 2016 107 080**
**US-A- 4 771 792**    **US-A1- 2016 143 625**

EP 3 308 714 B1

**Description**

Technical Field

The present invention relates to an ultrasonic imaging apparatus, and particularly to an ultrasonic imaging apparatus having the function of calculating hardness of an organ and a method for measuring the hardness.

Background Art

To perform early diagnosis and progress observation of a heart disease, information on pressure in the left atrium and the left ventricle is an important index. As an approach for noninvasive measurement of intracardiac pressure, NPL 1 proposes a method for estimating the hardness of the heart muscle from the natural frequency of the vibration of the left ventricle and further estimating the intracardiac pressure (PTL 1, NPL 1). To calculate the natural frequency of the vibration of the left ventricle, the technology described in NPL 1 uses approximation of the left ventricle to be a spherical shell, but the technology described in PTL 1 obtains information on the shape of the heart and improve the precision in calculation of the natural frequency in consideration of a shape factor.

Changes in the intracardiac pressure (cardiac failure) can be broadly classified into a change resulting from a decrease in the cardiac function and a change resulting from a burden acting on the heart due to congestion across the body although the cardiac function is normal. The different causes require different treatments. That is, depending on the cause, it is necessary to choose administration of a cardiac stimulant that enhances the cardiac function or administration of a diuretic agent that eliminates congestion, but it is difficult for an approach based on a change in the intracardiac pressure to determine a cause of cardiac failure.

Citation List

Patent Literature

PTL 1: International Publication No. WO2011/102221

Non Patent Literature

NPL 1: Honda, H et al. Am J Physiol Heart Circ Physiol 266: H881-H890 (1994)

EP 2 537 469 A1 relates to an ultrasonic imaging device and information processing device including an ultrasonic probe that transmits and receives ultrasonic waves to and from the heart, a signal-processing section that processes reflected echo signals received by the ultrasonic probe and a display section that displays the results of signal-processing as an image, wherein an input section is provided for setting a predetermined point on the image displayed on the display section.

Summary of Invention

Technical Problem

To solve the problem, for example, it is conceivable that knowledge of the degree of congestion in the kidney, which deeply relates to the heart, is likely to allow identification of the aforementioned causes of a change in intracardiac pressure. Applying the method of related art for estimating the intracardiac pressure to the diagnosis of the state of congestion in the kidney brings up several problems. First, the approach of related art uses approximation of the left atrium to be a spherical shell or an elliptical shell, but the shape approximation cannot be applied to the kidney. Further, in the approach of related art, the natural frequency is estimated in a predetermined vibration mode of the heart, but there are a variety of vibration modes, and a vibration mode appropriate for hardness measurement varies in accordance with a target organ.

An object of the invention is to provide an ultrasonic imaging apparatus and method capable of measuring the hardness of an organ in consideration of the vibration mode of the organ.

Solution to Problem

To achieve the object described above, an ultrasonic imaging apparatus and a method for calculating hardness are provided according to the features of the independent claims. The dependent claims relate to advantageous embodiments of the invention. An ultrasonic imaging apparatus according to the invention measures vibration on the basis of an ultrasonic echo signal and identifies a vibration mode on the basis of a result of the measurement. Having identified the vibration mode, the ultrasonic imaging apparatus calculates a natural frequency in the vibration mode and estimates the hardness of the organ.

Advantage of Invention

According to the invention, since a vibration mode appropriate for estimation of the hardness of an organ is identified in correspondence with the organ and the hardness is then estimated, the hardness or the in-organ internal pressure derived from the hardness can be measured with increased precision. The invention is applicable not only to the kidney but to an arbitrary organ. However, for example, adapting the invention to measurement of the hardness of the kidney allows provision of diagnosis information leading to identification of a cause of a heart failure difficult to locate.

Brief Description of Drawings

[Fig. 1] Fig. 1 shows the overall configuration of an ultrasonic imaging apparatus to which the invention is applied.

[Fig. 2] Fig. 2 shows an embodiment of hardness measurement.

[Fig. 3] Fig. 3 shows the spectrum of vibration of an organ.

[Fig. 4] Fig. 4 shows results of a simulation for determining a constant (vibration constant) used in hardness calculation.

[Fig. 5] Figs. 5 (a) and 5 (b) each show a vibration mode of the kidney.

[Fig. 6] Fig. 6 shows the procedure of processes carried out by a signal processing section in a first embodiment.

[Fig. 7] Fig. 7 shows an example of size measurement.

[Fig. 8] Fig. 8 describes speed measurement locations.

[Fig. 9] Fig. 9 shows an example of the timing chart of vibration excitation and ultrasonic wave transmission.

[Fig. 10] Fig. 10 describes vibration excitation in M-mode imaging.

[Fig. 11] Fig. 11 shows the procedure of vibration mode identification in the first embodiment.

[Fig. 12] Fig. 12 diagrammatically shows RF signals measured at two locations.

[Fig. 13] Fig. 13 describes the vibration mode identification in the first embodiment, Fig. 13(a) showing a spectrum sum and a spectrum of a speed sum and Fig. 13 (b) showing a difference spectrum.

[Fig. 14] Figs. 14(a) to 14(c) each show an example of display on a display section.

[Fig. 15] Figs. 15(a) to 15(d) each show a variation of an echo signal measuring method.

[Fig. 16] Fig. 16 shows the procedure of the vibration mode identification in a second embodiment.

[Fig. 17] Fig. 17 describes the vibration mode identification in the second embodiment, Fig. 17(a) showing changes in speeds of two points and Fig. 17(b) showing the phase difference between spectra at the two points.

Description of Embodiments

An embodiment of an ultrasonic imaging apparatus according to the invention will be described below.

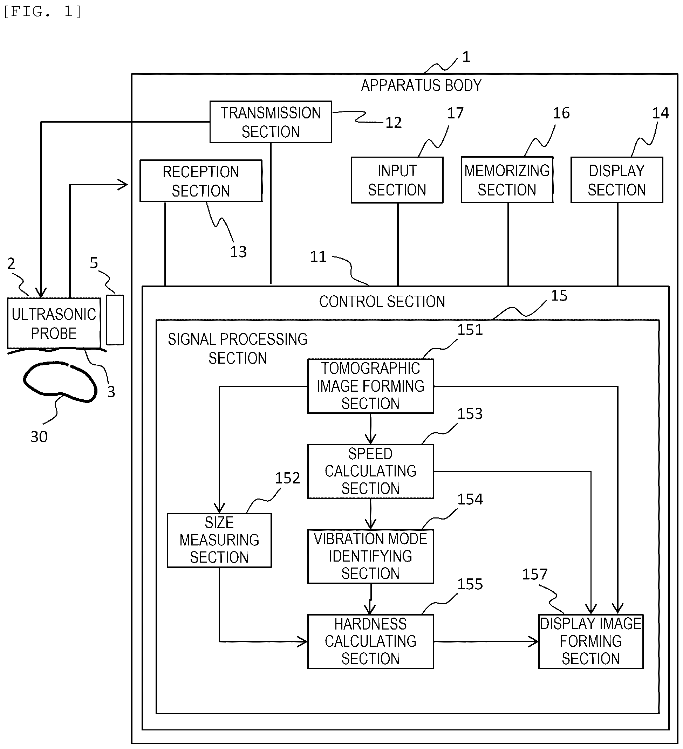

The embodiment of an ultrasonic imaging apparatus to which the invention is applied will first be described with reference to Fig. 1. The ultrasonic imaging apparatus (ultrasonic wave transmitting/receiving apparatus) shown in Fig. 1 includes an ultrasonic probe 2, an apparatus body 1, which produces an ultrasonic image while controlling drive operation of the ultrasonic probe (hereinafter simply referred to as probe) 2, and a vibration exciter 5, which excites predetermined vibration of a subject 3. The vibration exciter 5 may be so configured that the probe 2 also serves as the vibration exciter 5 or may be an external vibration exciter separate from the probe 2 and is hereinafter collectively referred to as a vibration exciting section 5.

The probe 2 includes a large number of vibrators arranged one-dimensionally or two-dimensionally in the vicinity of a surface where the probe 2 comes into contact with the subject 3 and along the contact surface, and the probe 2 irradiates the subject 3 with an ultrasonic wave emitted from each of the vibrators in accordance with a signal sent from the apparatus body 1, receives reflection echo reflected off an organ 30 or any other object in the subject 3, and sends the received reflection echo in the form of an RF signal to the apparatus body 1.

The apparatus body 1 includes a transmission section 12, which transmits a transmission pulse electric signal to the ultrasonic probe 2, a reception section 13, which receives an ultrasonic wave (acoustic signal) that is a wave reflected off an inspection target, a signal processing section 15, which processes the signal received by the reception section 13 to create an image and performs a variety of types of computation, a control section 11, which controls the action of each portion of the apparatus body 1, and a memorizing section (memory) 16. The apparatus body 1 further includes an input section 17, including a keyboard, a pointing device, and other components that allow an inspector who operates the ultrasonic imaging apparatus to set conditions under which the ultrasonic imaging apparatus operates in the control section 11, and a display section 14, which displays a result of a process carried out by the signal processing section 15 and other pieces of information. The input section 17 and the display section 14 may be so disposed as to be close to each other and may function as a user interface via which the inspector interactively operates the apparatus body 1.

In the present specification, the functions of the probe 2, the transmission section 12, and the reception section 13 and the control section, which causes the sections described above to operate, are collectively called an ultrasonic measurement section 10.

The action of the ultrasonic measurement section 10 is the same as the action of a typical ultrasonic wave transmitting/receiving apparatus and will be briefly described as follows : A beam former of the transmission section 12 first sends a transmission pulse electric signal to the probe 2 via a digital/analog (D/A) converter, and the probe 2 issues an ultrasonic wave (acoustic signal) toward an inspection target. The acoustic signal reflected in the course of propagation of the acoustic signal in the inspection target is received with the probe 2, converted into an electric signal, and sent as reception data via an A/D converter that is not shown to a reception beam former of the reception section 13. The reception beam former sums signals received with a plurality of elements in consideration of a temporal delay for which the transmission is performed at the time of transmission. The reception signal having undergone the summation then undergoes attenuation correction and other types of processing in a correction section that is not shown and sent in the form of RF data to the signal processing section 15. The signal processing section 15 uses the RF data measured by the measurement section 10 to create an image and calculate the hardness of the target organ.

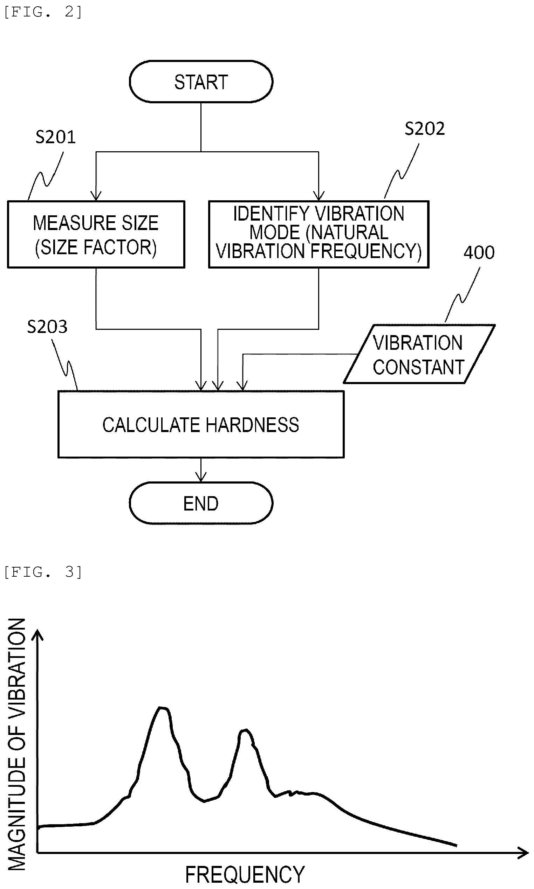

The hardness measurement in the present embodiment uses a pre-calculated characteristic of the vibration mode of the organ to identify a measurement location where the vibration mode can be clearly extracted. The signal processing section 15 identifies the vibration mode from measured data measured at the identified location and calculates the hardness of the organ in the identified vibration mode. In the hardness measurement, a basic process carried out by the signal processing section 15 includes size measurement S201, vibration mode identification S202, and hardness calculation S203, as shown in Fig. 2. To carry out the processes described above, the signal processing section 15 in the present embodiment includes a tomographic image forming section 151, a size measuring section 152, a tissue speed calculating section (hereinafter simply referred to as speed calculating section) 153, a vibration mode identifying section 154, and a hardness calculating section 155. The signal processing section 15 can further include a display image crating section 157, which creates an image that allows the display section 131 to display a result of a process.

The tomographic image forming section 151 creates a morphologic image of a cross section irradiated with the ultrasonic wave. The size measuring section 152 measures the size of the target organ, such as the diameter along the major axis and the diameter along the minor axis by using the morphologic image created by the tomographic image forming section 151 (S201). The speed calculating section 153 calculates the speed of a predetermined position on the organ in the morphologic image by using a temporal change in the position. The vibration mode identifying section 154 identifies a vibration mode used in the hardness calculation by using the difference between speeds of at least two locations calculated by the speed calculating section 153 (S202). The hardness calculating section 155 calculates the hardness of the target organ by using the speeds calculated by the speed calculating section 153, the natural vibration frequency in the vibration mode identified by the vibration mode identifying section 154, and a constant (constant relating to shape) 400 determined in advance (S203). The display image creating section 157 creates an image for displaying the tomographic image created by the tomographic image forming section 151, the hardness calculated by the hardness calculating section 155, and other pieces of information in a predetermined display format. The display image creating section 157 further creates, for example, a UI that allows the inspector to input an instruction and a condition necessary for control and signal processing via the input section 17.

In the process described above, the size measuring section 152 produces information on the size by using the morphologic image created by the tomographic image forming section 151. Instead, an image acquired by an imaging apparatus other than the ultrasonic imaging apparatus, for example, an X-ray imaging apparatus is captured, and the size can be measured on the basis of the captured image. In this case, the tomographic image formation is not essential.

The function of each of the functional sections described above that form the signal processing section 15 can be achieved by a processor (CPU (central processing unit) or GPU (graphics processing unit)) that executes a program stored in the memory. Instead, part or entirety of the functions may be achieved by hardware, such as an ASIC (application specific integrated circuit) and an FPGA (field-programmable gate array).

The control section 11 is primarily formed of a CPU and controls the actions of the transmission section 12, the reception section 13, and the signal processing section 15. In the case where part or entirety of the functions of the signal processing section 15 is achieved by a CPU, the CPU may also serve as the CPU that forms the control section 11.

The memorizing section 16 stores numerical values, such as parameters necessary for computation in the signal processing section 15, and data in the course of the computation or a result of the computation, and the memorizing section 16 may include an internal memory as well as an external storage device. In the present embodiment, the memorizing section 16 stores constants (Fig. 2, reference character 400) used in the calculation performed by the hardness calculating section 155.

On the basis of the configuration described above, an embodiment of the hardness measurement performed by the ultrasonic imaging apparatus according to the present embodiment will be described below.

<First embodiment>

In the present embodiment, the vibration mode and the vibration frequency thereof are identified on the basis of data measured at at least two locations that move differently in accordance with the vibration mode, and the hardness of the target organ is calculated by using the size of the target organ and a shape-relating constant (vibration constant) specified in advance on a vibration mode basis.

The relationship between the vibration constant determined in advance by calculation and the vibration mode will first be described. The vibration constant used herein is a constant determined by the shape of an organ and having a value that varies in accordance with the vibration mode. In general, when predetermined vibration is applied to an organ, frequency analysis of the vibration of the organ provides a spectrum having peaks at different frequencies determined in accordance with the vibration mode, as shown in Fig. 3. Fig. 3 diagrammatically shows a spectrum having two peaks, and the height of each of the peaks and the number of peaks vary in accordance with an organ that vibrates. The peaks correspond to a plurality of vibration modes having different natural frequencies.

The present inventors have studied an organ having a shape that approximates to a spherical or elliptical shell, such as the heart, and have shown that the hardness E of the organ can be expressed by Expression (1) by using a frequency fn of a vibration mode n (PTL 1).
[Expression 1]

$$E = \rho C_n r^2 f_n^2 \quad (1)$$

where $\rho$ represents the density of the organ, and r represents the diameter of the organ. Cn represents a constant (vibration constant) determined by the shape of the organ and have a value that varies in accordance with the vibration mode.

Expression (1) has been extended to an organ having lengths in the major and minor axis directions different from each other, such as the kidney, and a simulation has been performed by using a size factor L, as a size factor in place of "r", determined by the length in the major axis direction or the combination of the length in the major axis direction and the length in the minor axis direction. The result of the simulation shows that Expression (2) holds true also for an organ having a shape that does not approximate to a spherical or elliptical shell. In Expressions (1) and (2), the vibration constant is expressed by the same character Cn, but Cn has different values in Expressions (1) and (2).
[Expression 2]

$$E = \rho C_n L^2 f_n^2 \quad (2)$$

That is, the hardness E is proportional to the square of the vibration frequency, $\rho$ is a constant fixed when an organ is determined, and L is a constant measurable from the morphology of the organ. Therefore, once the vibration mode n is determined, the hardness E can be calculated by using the vibration constant Cn and the frequency fn of the vibration mode n.

The vibration constant Cn can be calculated in a simulation by substituting an imaginary value for E into Expression (3), which is a deformation of Expression (2).
[Expression 3]

$$C_n = \frac{E}{\rho L^2 f_n^2} \quad (3)$$

That is, a human kidney having specified E, $\rho$, and L and caused to vibrate is simulated in each vibration mode to calculate the natural frequency of the kidney. The vibration frequency fn in each vibration mode is thus calculated. The calculated vibration frequency fn is substituted into Expression (3) to calculate Cn.

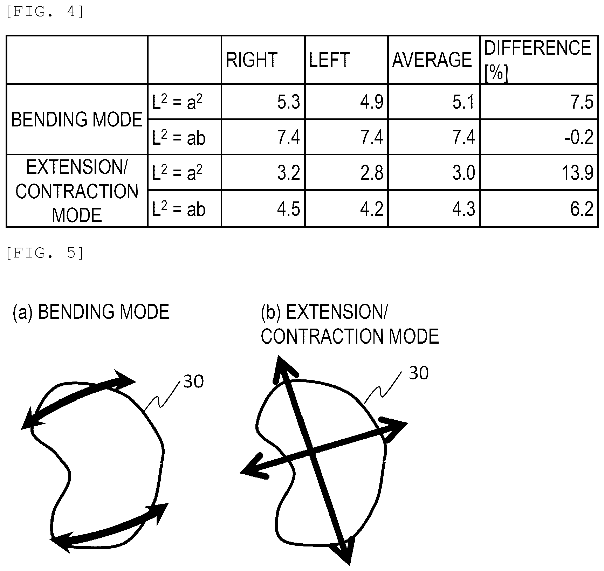

Fig. 4 shows results of an actual simulation performed on the right and left kidneys. The values in the table shown in Fig. 4 are calculated Cn values. It has been assumed that the simulated vibration modes are the following two modes specific to the kidney: a bending mode shown in Fig. 5 (a); and an expansion/contraction mode shown in Fig. 5(b). The size factor L is assumed to be calculated as follows: L=a² or L=a×b, where a represents the length of the kidney in the major axis direction, and b represent the length of the kidney in the minor axis direction. The calculated Cn values for the right kidney well coincide with those for the left kidney irrespective of the form of the size factor or the type of the vibration mode, whereby Expression (2) has been found to be valid.

The Cn values derived from the simulation are stored along with the vibration mode and the size factor in the memorizing section 16. The simulation can be performed by using a computer different from the ultrasonic imaging apparatus according to the present embodiment, and results of the simulation may be stored in the memorizing section 16 of the ultrasonic imaging apparatus. Instead, a simulator that performs the calculation described above can be provided as part of the signal processing section 15 of the ultrasonic imaging apparatus.

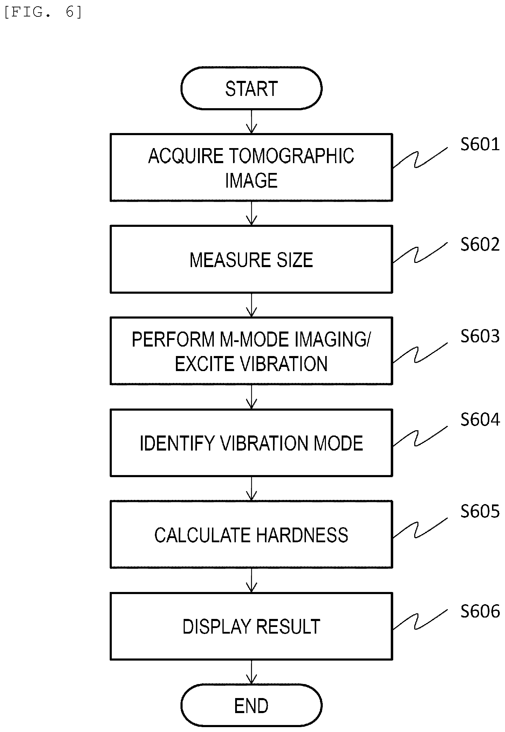

The procedure of the hardness measurement performed by the signal processing section 15 will next be described with reference to a case where the organ is the kidney. Fig. 6 shows an overview of the procedure.

The tomographic image forming section 151 first creates a morphologic image of the kidney by using a signal measured by the measurement section 10 and representing measurement of the whole kidney as an imaging target (S601). The size measuring section 152 then measure the size of the kidney by using the morphologic image created by the tomographic image forming section 151 (S602) to acquire the size factor L. To determine the vibration mode, imaging for measuring speeds of two points on the target organ is performed (S603), and the speed calculating section 153 calculates the speeds of the two points. The vibration mode identifying section 154 then determines the vibration mode n and the vibration frequency fn by using a change in the speeds of the two points (S604). The hardness calculating section 155 calculates the hardness E in accordance with Expression (2) by using the vibration frequency fn of the vibration mode n calculated by the vibration mode identifying section 154, the vibration constant Cn for the vibration mode n read from the memorizing section 16, and the size factor L (S605). The display image creating section 157 creates a result-showing image in a predetermined display form and causes the display section 14 to display the image (S606). In Fig. 6, the processes in S601 and S602 relating to the size measurement and the processes in S603 and S604 relating to the vibration mode identification are sequentially arranged, but the order of the processes may be reversed, or part of the processes can be concurrently carried out.

The process carried out by each of the sections of the signal processing section 15 will be specifically described below.

To perform the size measurement, the ultrasonic measurement section 10 first causes the transmission section 12 and the reception section 13 to operate and receives an ultrasonic signal under the control of the control section 11. The tomographic image forming section 151 creates a B-mode image (tomographic image) from the received ultrasonic signal (S601). A method of the imaging method and the creation of a B-mode image are the same as those in a known ultrasonic imaging apparatus.

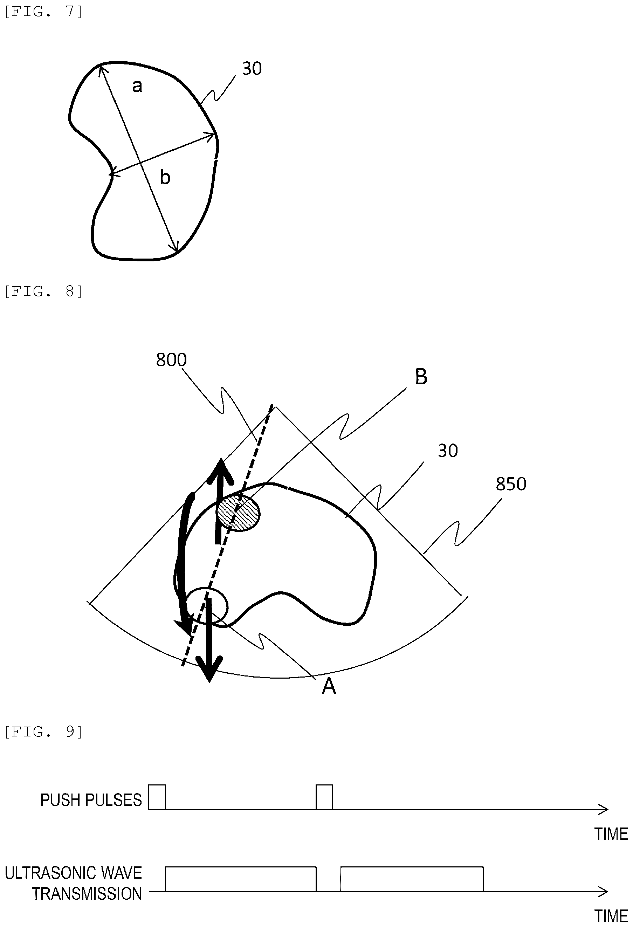

The size measuring section 152 measures the size of the inspection target, the lengths of the kidney in the major and minor axes thereof in the description, on the basis of data on the image (S602). An approach to the size measurement may be a manual approach involving the inspector or may be an automatic approach involving no inspector. In the former case, the display image creating section 157 causes the display section 14 to display the tomographic image and accepts the inspector's instruction of an on-screen start point and end point in the length measurement. The size measuring section 152 calculates the distance between the two points on the basis of the coordinates of the accepted two points in the major axis direction and two points in the minor axis direction and determines the length a in the major axis direction and the length b in the minor axis direction, as shown in Fig. 7. In the latter case, the difference in luminance in the image is used to extract the contour of the organ, and the lengths of the extracted contour in the major and minor axis directions are determined. For example, the greatest diameter of the contour may be determined as the length in the major axis direction, and the axis passing through the center of the major axis between the two points and perpendicular to the major axis may be determined to be the minor axis and the length along the thus determined minor axis may be determined as the length in the minor axis direction. Instead, an oblong that circumscribes the contour of the morphologic image may be defined, and the length of the long sides of the oblong may be determined as the length in the major axis direction. The minor axis is the same as described above. The number of cross sections each of which forms a morphologic image is not limited to one, and a plurality of cross sections created by changing the position where the ultrasonic probe 2 comes into contact with the subject 3 and the angle at which the ultrasonic probe 2 comes into contact with the subject 3 may be used to calculate the lengths in the major and minor axis directions. The thus calculated lengths in the major and minor axis directions are memorized in the memorizing section 16 and used in the hardness calculation (S605).

In the imaging step S603, the measurement section 10 first performs imaging (S603) for obtaining information necessary for the vibration mode identification (S604). The measurement section 10 acquires a one-line image passing through predetermined two points on the kidney, and the tomographic image forming section 151 creates an M-mode image. The line passing through the predetermined two points (ultrasonic beam scan line) is preferably a line 800, which passes through a location where a difference between the two representative vibration modes of the kidney, the bending mode and the expansion/contraction mode, is shown, specifically, passes through a point in the vicinity of a deep protruding site A of the kidney, as shown in Fig. 8. Such two points A and B may, for example, be specified by the inspector with a tomographic image 850 acquired in step S601 displayed on the display section 14 or can, for example, be automatically set by using an image recognition technology, as in the automatic size measurement described above.

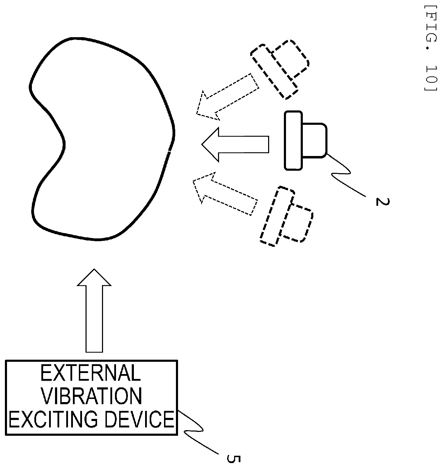

In the high-speed M-mode imaging, the vibration exciting section 5 is activated to excite vibration of the kidney. The action of the vibration exciting section 5 may be controlled by the control section 11 or may be controlled separately from the ultrasonic imaging apparatus. The frequency of the vibration excited by the vibration exciting section 5 is preferably the frequency of white-noise-like excited vibration that completely covers frequency bands of vibration to be observed and falls within a range from about 1 to 300 MHz. The frequency of an ultrasonic wave used to perform the M-mode imaging falls within a range from about 1 to 30 MHz, which is used to perform typical ultrasonic imaging. The vibration excited by the vibration exciting section 5 may be push pulses having a duration of about 300 μs. Fig. 9 shows an example of the relationship between the vibration excited by the vibration exciting section 5 and the ultrasonic pulses. In this example, the push pulses and the ultrasonic wave pulse irradiation in the high-speed M-mode imaging are repeated in a fixed cycle, and a plurality of RF signals are acquired at different points of time within each of the ultrasonic wave pulse irradiation periods. The vibration excitation direction is not limited to a specific direction. In the case where the ultrasonic probe 2 excites vibration, the orientation of the ultrasonic probe 2 is so adjusted as to excite vibration in a desired direction, as shown in Fig. 10. In the case where an external vibration exciting device is used, in which the flexibility is increased, the vibration excitation direction can be selected as appropriate in accordance with the position and orientation of the organ.

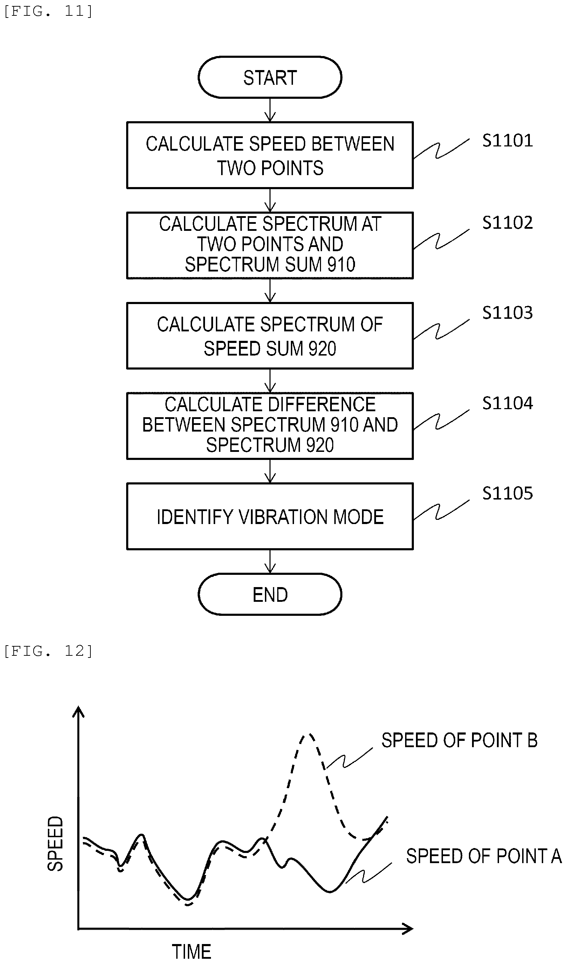

The vibration mode identifying section 154 then identifies the vibration mode by using a signal produced in the high-speed M-mode imaging described above (S604). Fig. 11 shows the vibration mode identification in detail. The speed calculating section 153 first detects the phase difference between the waveforms of M-mode RF signals measured at different points of time and calculates the speed of a point that is observation target (S1101). The phase difference can be detected by using autocorrelation or improved autocorrelation frequently used in ultrasonic Doppler, and the speed calculating section 153 is equipped with an algorithm that achieves the phase difference detection. Since the phase difference corresponds to the distance over which the point that is observation target has moved in a certain period (difference between points of time when RF signals are measured), the speed can be determined by dividing the difference by the period. The speed is calculated at each of the two points A and B shown in Fig. 8, and a temporal change in the speed is determined.

Fig. 12 diagrammatically shows changes in the thus calculated speeds of the two points (A, B). In Fig. 12, the solid line represents the speed of the point A (deep protruding site of kidney), and the dotted line represents the speed of the point B. The two points move at roughly the same speed for some periods and move at totally different speeds for other periods, as seen from Fig. 12, which means that in the bending mode shown in Fig. 5(a), the two points move in roughly the same manner in the scan line direction (line passing through two points), whereas in the expansion/contraction mode shown in Fig. 5(b), for example, when the kidney expands or contracts in the scan line direction, the points A and B move in opposite directions.

The vibration mode identifying section 154 then enhances or attenuates one of the vibration modes and identifies the vibration mode by using the difference between the changes in the speeds of the two points calculated by the speed calculating section 153. Specifically, frequency analysis based on Fourier transform or any other type of operation is performed data on the change in the speed of each of the points to produce a spectrum of the speed of the point, and the sum of the spectra is determined (S1102). A peak that appears in a spectrum corresponds to a vibration mode, as described above, and the spectrum at each of the points contains peaks corresponding to a plurality of vibration modes. The magnitude of a peak varies in accordance with the position of each of the points. That is, the following two types of spectrum are obtained: a spectrum showing the bending mode more clearly than the expansion/contraction mode in accordance with the position of each of the points; and a spectrum showing the expansion/contraction mode more clearly than the bending mode in accordance with the position of each of the points. Summing the spectra allows increase in the magnitude of the vibration of each of the vibration modes.

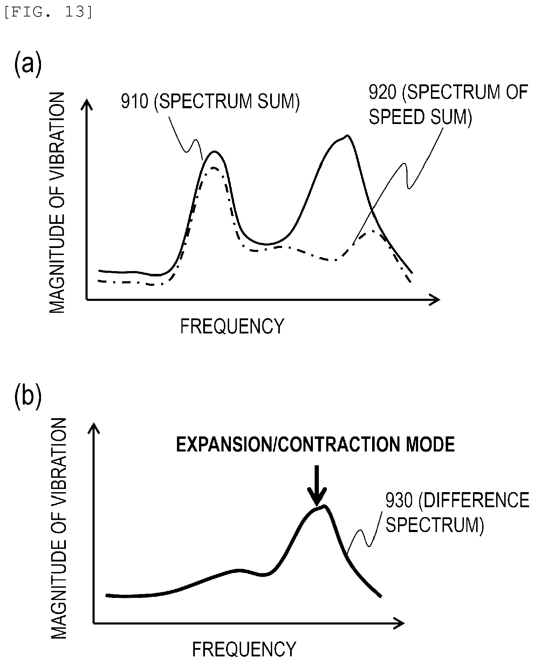

The vibration mode identifying section 154 sums the speeds of the two points (speed sum) and calculates the spectrum of the speed sum (S1103). The speeds of the two points behave as follows: The two points move roughly in synchronization with each other in the bending mode; and the motions of the two points have opposite phases in the expansion/contraction mode, as shown in Fig. 12. Therefore, in the speed sum, the value of the speed in the bending mode is roughly doubled and hence enhanced, whereas the value of the speed is attenuated in the expansion/contraction mode. Therefore, in the spectrum of the speed sum, the peak in the bending mode dominates. Fig. 13(a) diagrammatically shows the "spectrum sum" 910 produced in S1102 and the "spectrum of speed sum" 920 produced in S1103. In Fig. 13(a), the solid line represents the "spectrum sum," and the dashed line represents the "spectrum of speed sum." The processes in S1102 and S1103 can be carried out in any order.

As the last step, the vibration mode identifying section 154 calculates the difference between the "spectrum sum" and the "spectrum of speed sum" (S1104). In the resultant difference spectrum 930, only the peak in one of the vibration modes (expansion/contraction mode in the description) remains, as shown in Fig. 13(b). The difference spectrum allows determination of the vibration mode and the natural vibration frequency thereof (S1105). That is, the vibration mode n and the natural vibration frequency $f_n$ in Expression (2) are identified.

Further, in a case where the two vibration modes each have a peak, as shown in Fig. 13, identification of one of the two vibration modes (expansion/contraction mode in the description) allows identification of the other (bending mode), whereby the natural vibration frequencies in the vibration modes can be identified. Further, in the above description, the vibration mode to be identified first is the expansion/contraction mode. Instead, the two points on the organ can be so selected that they move in the direction opposite the direction described above.

Once the vibration mode is identified as described above, the hardness calculating section 155 calculates the hardness E of a kidney 30, which is the measurement target, by using the vibration constant $C_n$ of the vibration mode n memorized in the memorizing section 16, the size factor L measured by the size measuring section 152 and determined by the lengths a and b of the kidney 30 in the major and minor axis directions thereof, and the natural vibration frequency $f_n$ identified by the vibration mode identifying section 154 (S605). In this case, $f_n$ and $C_n$ of a plurality of vibration modes can be used to calculate the respective hardness E for improvement in precision of the calculation and ascertainment of the degree of accuracy of the measurement.

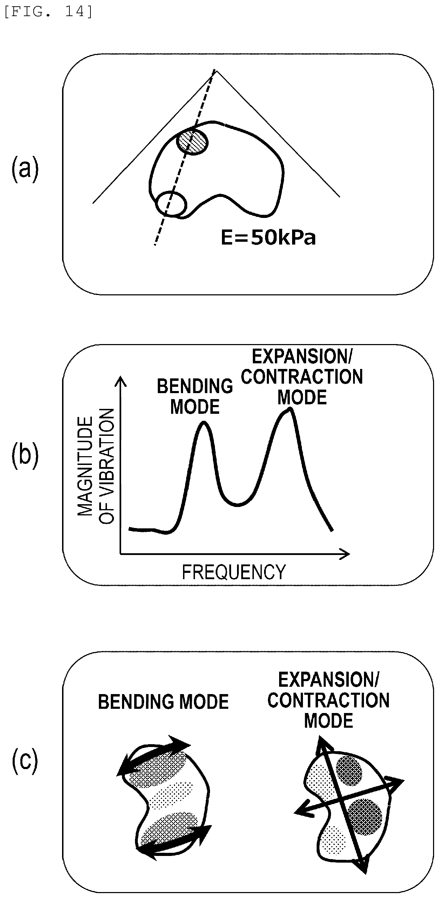

The display image creating section 157 can display results of the calculation performed by the vibration mode identifying section 154 and the hardness calculating section 155 on the display section 14 in a variety of display forms (S606). Fig. 14 shows several display examples. Fig. 14(a) shows that calculated hardness E of the kidney is displayed in the form of a numeral. In the illustrated example, the hardness E is so displayed in the form of a numeral as to be superimposed on the tomographic image of the kidney. Further, the tomographic image of the kidney may be combined with the position of the scan line and the measured two points and further with arrows diagrammatically indicating the vibration mode and other pieces of information. Fig. 14(b) shows that a spectrum ("spectrum sum," for example) is displayed, and each peak of the spectrum is labeled with a corresponding vibration mode in the form of letters.

Fig. 14(c) shows that the phase is displayed on a frequency basis. In the display example, it is assumed that a spectrum has been derived for each of a plurality of regions of the kidney. For example, in the bending mode shown on the left in the figure, in which the kidney is divided into three regions, the regions on the opposite sides change in the same phase, and the phase of these regions is opposite the phase of the central region. In the expansion/contraction mode shown on the right in the figure, the upper and lower sides of the organ divided by a center line roughly perpendicular to the scan line change in opposite phases. A temporal change in the phase (ranging from $-\pi$ to $+\pi$) can be shown by using color codes, contour lines, or any other display method, whereby the motion of the kidney can be grasped in detail.

The display examples shown in Fig. 14 are not necessarily employed and can be arbitrarily combined with each other or can be combined with another piece of information. For example, the hardness of the heart may be measured by using a known method (method described in PTL 1, for example), and a result of the measurement may be displayed along with the hardness of the kidney side by side. Such a display image serves as an assistant image in diagnosis of a heart disease.

As described above, the ultrasonic imaging apparatus according to the present embodiment analyzes the speeds of locations where a difference between vibration modes of the displacement due to vibration occurs to identify the vibration mode and the frequency. Further, the constant (vibration constant) relating to the shape of an organ is determined in advance on a vibration mode basis, and the vibration constant in the identified vibration mode, the vibration frequency, and the size factor of the organ are used to calculate the hardness of the organ.

According to the present embodiment, the hardness can be calculated on the basis of identification of the vibration mode, whereby the precision of the hardness calculation can be improved. As a result, in the case where the kidney is the target organ, the state of congestion in the kidney can be estimated by an index in the form of the hardness, whereby a heart disease can be more accurately diagnosed.

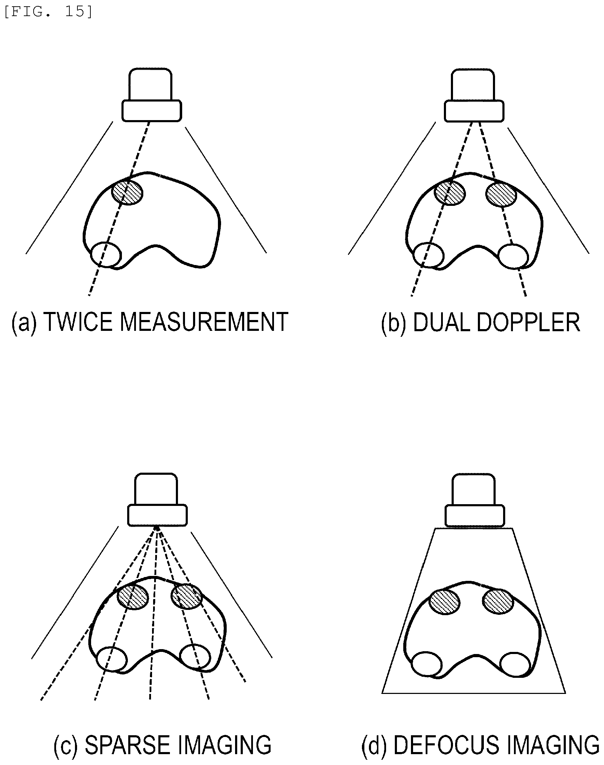

The present invention is not limited to the embodiment shown in the drawings having been referred to in the description and can be changed in a variety of manners. For example, Fig. 8 shows the case where two points on the kidney and one scan line passing through the two points are set, and the same measurement may be performed by using the line passing through two symmetrically positioned points where a similar vibration behavior is observed. In a case where the measurement is performed by using two or more lines, one-line measurement may be repeated (Fig. 15(a)), or in the case of an apparatus having a dual Doppler function that allows simultaneous irradiation along two scan lines, simultaneous two-line measurement may be performed (Fig. 15(b)). In a case where the measurement is performed by using three or more line, sparse imaging in which a transducer is so driven that thinning imaging is performed may be employed (Fig. 15(c)). Further, the measurement may be performed by using defocus imaging (Fig. 15(d)).

In the above description, the square of the length in the major axis direction ($a^2$) and the length in the major axis direction $\times$ the length in the minor axis direction ($a \times b$) are presented as examples of the shape factor L, but the shape factor is not specifically limited thereto and may be any constant that satisfies Expression (2), for example, the area of an organ.

The present embodiment has been described with reference to the case where the kidney is the measurement target organ. The present embodiment is, however, also applicable to a variety of organs that does not approximate to a spherical or elliptical shell in addition to the kidney.

<Second embodiment>

In the first embodiment, spectra of the speeds (spectrum sum and spectrum of speed sum) are used to identify the vibration mode. On the other hand, in the present embodiment, a spectrum phase difference is calculated, and the vibration mode is identified from the phase difference. That is, the vibration mode identifying section in the present embodiment identifies the vibration mode and the frequency in the vibration mode by using the phase difference between the spectra of the speeds of the two locations.

In the present embodiment, since the overview of the configuration of the signal processing section 15 and processes carried out by the signal processing section 15 is the same as that in the first embodiment, the processes in the present embodiment will be described with the aid of Fig. 6 used in the first embodiment. The tomographic image forming section 151 first creates a morphologic image of a target organ by using a reception signal measured by the measurement section 10 (S601), and the size measuring section 152 measures the size of the target organ (S602). In addition to the imaging (S601), the measurement section 10 selects at least two points where the vibration mode is likely to vary and performs the M-mode high-speed measurement (S603), and the speed calculating section 153 calculates the speeds of the two points (S604).

The vibration mode identifying section 154 identifies the vibration mode by using the speeds of the two points calculated by the speed calculating section 153.

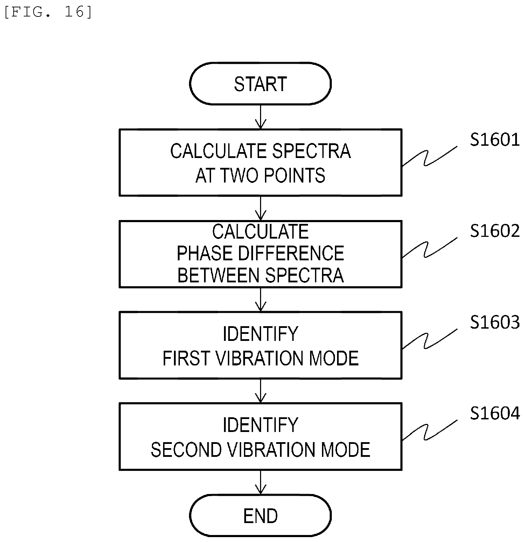

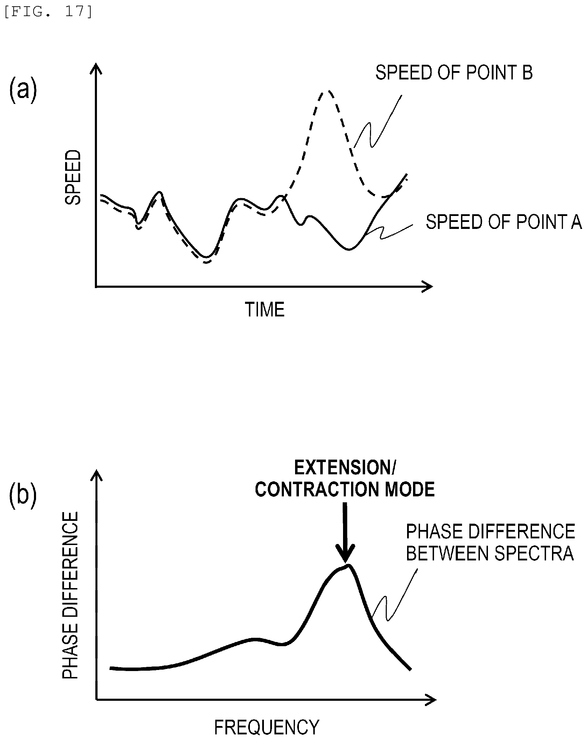

Fig. 16 shows the procedure of the processes carried out by the vibration mode identifying section 154. The vibration mode identifying section 154 first calculates the spectra of the speeds of the two points (S1601) and determines the phase difference between the two spectra (S1602). As described in the first embodiment, in the case of the speeds of the two points A and B, where there is a difference between the two representative vibration modes of the kidney, the bending mode and the expansion/contraction mode, as shown, for example, in Fig. 8, the phases in the two modes roughly coincide with each other in some periods, and they are roughly opposite each other in other periods (Fig. 17(a)). When the phases in the two modes roughly coincide with each other, the vibration in the bending mode is dominant, and when they are roughly opposite each other, the vibration in the expansion/contraction mode is dominant. The shift in the phase also appears in the spectra. Calculating the phase difference between the spectra therefore provides a peak in the expansion/contraction mode, in which a large phase difference between the spectra is provided, as shown in Fig. 17(b). The vibration mode identifying section 154 identifies the peak to be a peak in an expansion mode (first vibration mode) and identifies the frequency at the peak to be the vibration frequency in the expansion/contraction mode (S1603).

Out of two peaks (highest peak and peak showing second largest magnitude) present in the spectra determined from the speeds of the two points, the peak that does not correspond to the peak of the vibration frequency identified in S1603 is identified to be a compression mode (second vibration mode) and the vibration frequency in the compression mode is identified (S1604).

After the two vibration modes and vibration frequencies are identified, the hardness calculating section 155 calculates the hardness of the organ by using the vibration frequency fn, the size factor L, and the vibration constant Cn (S605), as in the first embodiment. In Fig. 16, two vibration modes are identified, and the hardness can be calculated even by using one vibration mode.

According to the present embodiment, appropriately setting a site where the speed measurement is made allows the vibration mode to be more readily identified than in the first embodiment. The variations described in the first embodiment are similarly applicable to the present embodiment.

Reference Signs List

1: Apparatus body, 2: Ultrasonic probe, 3: Subject, 10: Ultrasonic measurement section, 11: Control section, 12, Transmission section, 13: Reception section, 14: Display section, 15: Signal processing section, 16: Storage section (memory), 17: Input section, 1151: Tomographic image forming section, 152: Size measuring section, 153: Speed calculating section, 154: Vibration mode identifying section, 155: Hardness calculating section, 157: Display image creating section

Claims

1. An ultrasonic imaging apparatus comprising:

an ultrasonic measurement section (10) that includes an ultrasonic probe (2) and configured to measure an echo signal from a region containing a desired organ (30); and
a signal processing section (15) configured to perform computation by using the echo signal, the ultrasonic imaging apparatus **characterized in that**
the signal processing section (15) includes a speed calculating section (153) configured to calculate speeds of at least two locations on the organ (30) by using the echo signal,
a vibration mode identifying section (154) configured to analyze the speeds of the two locations to identify a plurality of vibration modes of the organ (30) and a vibration frequency in each of the vibration modes, and
a hardness calculating section (155) configured to calculate hardness of the organ (30) by using the vibration modes and the vibration frequencies identified by the vibration mode identifying section, a constant determined in -advance in relation to the organ, and information on a size of the organ (30),
wherein the organ (30) is a kidney, and the two locations are located on a side shifted from a center of a longitudinal length of the kidney toward one end thereof and at different ultrasonic depths.

2. The ultrasonic imaging apparatus according to claim 1, **characterized in that**
the signal processing section (15) further includes a size measuring section configured to calculate the information on a size of the organ based on a morphologic image of the organ (30).

3. The ultrasonic imaging apparatus according to claim 2, **characterized in that**
the morphologic image of the organ is an ultrasonic image formed from the echo signal measured by the ultrasonic measurement section (10).

4. The ultrasonic imaging apparatus according to claim 1, **characterized in that**
the constant is a value calculated by performing a simulation that uses a following expression and accepts an input of imaginary hardness as the hardness of the organ (30),

$$Cn = E'/\rho L^2 fn^2$$

wherein in the expression, E' represents the imaginary hardness, $\rho$ represents a density of the organ, Cn represents a constant in a vibration mode n wherein n is an integer greater than or equal to 1, the same holds true for the following n, L represents the size information, and fn represents a natural frequency in the vibration mode n.

5. The ultrasonic imaging apparatus according to claim 1, **characterized in that**
the ultrasonic imaging apparatus further comprises a memorizing section configured to memorize the constant.

6. The ultrasonic imaging apparatus according to claim 1, **characterized in that**
the vibration mode identifying section (154) is configured to identify the vibration modes and the frequencies in the vibration modes by using a difference between a spectrum of a sum of the speeds of the two locations and a sum of spectra at the two locations.

7. The ultrasonic imaging apparatus according to claim 1, **characterized in that**
the vibration mode identifying section (154) is configured to identify the vibration modes and the frequencies in the vibration modes by using a phase difference between spectra of the speeds of the two locations.

8. The ultrasonic imaging apparatus according to claim 1, **characterized in that**
the information on the size of the organ (30) includes any one of a size of the organ along a major axis thereof, a size of the organ (30) along a minor axis thereof, a volume of the organ, and an area of a predetermined cross section of the organ.

9. The ultrasonic imaging apparatus according to claim 1, **characterized in that**
the plurality of vibration modes include a bending mode and an expansion/contraction mode.

10. The ultrasonic imaging apparatus according to claim 1, **characterized in that**
the hardness calculating section (155) is configured to calculate the hardness by using a following expression:

$$E = \rho C n L^2 F n^2$$

wherein in the expression, E represents the hardness, $\rho$ represents a density of the organ, Cn represents a constant in a vibration mode n, L represents the size information, and fn represents a natural frequency in the vibration mode n.

11. The ultrasonic imaging apparatus according to claim 1, **characterized in that**
the echo signal used by the speed calculating section (153) is an echo signal measured in a situation in which a vibration exciter is configured to excite vibration of the organ.

12. The ultrasonic imaging apparatus according to claim 11, **characterized in that**
the ultrasonic probe (2) is also configured to serve as the vibration exciter.

13. The ultrasonic imaging apparatus according to claim 1, **characterized in that**
the ultrasonic imaging apparatus further comprises a display image creating section (157) configured to cause a display device to display a result calculated by the hardness calculating section (155).

14. A method for calculating hardness of an organ by using an ultrasonic reception signal received from the organ (30) by transmitting an ultrasonic signal to the organ (30) caused to vibrate, **characterized in that** the method comprises:

   calculating speeds of at least two locations on the organ (30) by using the ultrasonic reception signal,
   analyzing the speeds of the two locations to identify a plurality of vibration modes of the organ (30) and a vibration frequency in each of the vibration modes, and
   calculating hardness of the organ by using the identified vibration modes and vibration frequencies, information on a size of the organ (30), and a vibration constant calculated in advance for each of the plurality of vibration modes, wherein the organ is a kidney and the two locations are located on a side shifted from a center of a longitudinal length of the kidney toward one end thereof and at different ultrasonic depths.

**Patentansprüche**

1. Ultraschallbild-Erzeugungsvorrichtung, die Folgendes umfasst:

   einen Ultraschallmessabschnitt (10), der eine Ultraschallsonde (2) enthält und konfiguriert ist, ein Echosignal aus einem Bereich, der ein gewünschtes Organ (30) enthält, zu messen; und einen Signalverarbeitungsabschnitt (15), der konfiguriert ist, eine Berechnung unter Verwendung des Echosignals durchzuführen, wobei die Ultraschallbild-Erzeugungsvorrichtung **da-**

**durch gekennzeichnet ist, dass**
der Signalverarbeitungsabschnitt (15) Folgendes enthält:

einen Geschwindigkeitsberechnungsabschnitt (153), der konfiguriert ist, Geschwindigkeiten von mindestens zwei Orten auf dem Organ (30) unter Verwendung des Echosignals zu berechnen,
einen Schwingungsmodus-Identifizierungsabschnitt (154), der konfiguriert ist, die Geschwindigkeiten der zwei Orte zu analysieren, um mehrere Schwingungsmodi des Organs (30) und eine Schwingungsfrequenz in jedem der Schwingungsmodi zu identifizieren, und
einen Härteberechnungsabschnitt (155), der konfiguriert ist, eine Härte des Organs (30) unter Verwendung der Schwingungsmodi und der Schwingungsfrequenzen, die durch den Schwingungsmodus-Identifizierungsabschnitt identifiziert wurden, einer Konstante, die im Voraus in Bezug auf das Organ bestimmt wurde, und Informationen über eine Größe des Organs (30) zu berechnen, wobei
das Organ (30) eine Niere ist und die zwei Orte sich auf einer Seite, von einem Mittelpunkt einer Längsausdehnung der Niere zu einem ihrer Enden verschoben und bei zwei verschiedenen Ultraschalltiefen befinden.

2. Ultraschallbild-Erzeugungsvorrichtung nach Anspruch 1, **dadurch gekennzeichnet, dass**
der Signalverarbeitungsabschnitt (15) ferner einen Größenmessabschnitt umfasst, der konfiguriert ist, die Informationen über eine Größe des Organs auf der Grundlage eines morphologischen Bilds des Organs (30) zu berechnen.

3. Ultraschallbild-Erzeugungsvorrichtung nach Anspruch 2, **dadurch gekennzeichnet, dass**
das morphologische Bild des Organs ein Ultraschallbild ist, das aus dem Echosignal, das durch den Ultraschallmessabschnitt (10) gemessen wurde, gebildet wird.

4. Ultraschallbild-Erzeugungsvorrichtung nach Anspruch 1, **dadurch gekennzeichnet, dass**
die Konstante ein Wert ist, der durch Durchführen einer Simulation, die den folgenden Ausdruck verwendet und eine Eingabe einer imaginären Härte als die Härte des Organs (30) annimmt, berechnet wird:

$$Cn = E' / \rho L^2 fn^2,$$

wobei
im Ausdruck E' die imaginäre Härte repräsentiert, $\rho$ eine Dichte des Organs repräsentiert, Cn eine Konstante in einem Schwingungsmodus n repräsentiert, wobei n eine ganze Zahl größer oder gleich 1 ist, wobei dasselbe für das folgende n zutrifft, L die Größeninformationen repräsentiert und fn eine Eigenfrequenz im Schwingungsmodus n repräsentiert.

5. Ultraschallbild-Erzeugungsvorrichtung nach Anspruch 1, **dadurch gekennzeichnet, dass**
die Ultraschallbild-Erzeugungsvorrichtung ferner einen Speicherabschnitt umfasst, der konfiguriert ist, die Konstante zu speichern.

6. Ultraschallbild-Erzeugungsvorrichtung nach Anspruch 1, **dadurch gekennzeichnet, dass**
der Schwingungsmodus-Identifizierungsabschnitt (154) konfiguriert ist, die Schwingungsmodi und die Frequenzen in den Schwingungsmodi unter Verwendung einer Differenz zwischen einem Spektrum einer Summe der Geschwindigkeiten der zwei Orte und einer Summe von Spektren bei den zwei Orten zu identifizieren.

7. Ultraschallbild-Erzeugungsvorrichtung nach Anspruch 1, **dadurch gekennzeichnet, dass**
der Schwingungsmodus-Identifizierungsabschnitt (154) konfiguriert ist, die Schwingungsmodi und die Frequenzen in den Schwingungsmodi unter Verwendung einer Phasendifferenz zwischen Spektren der Geschwindigkeiten der zwei Orte zu identifizieren.

8. Ultraschallbild-Erzeugungsvorrichtung nach Anspruch 1, **dadurch gekennzeichnet, dass**
die Informationen über die Größe des Organs (30) eine Größe des Organs entlang seiner Hauptachse, eine Größe des Organs (30) entlang seiner Nebenachse, ein Volumen des Organs und eine Fläche eines vorgegebenen Querschnitts des Organs enthalten.

9. Ultraschallbild-Erzeugungsvorrichtung nach Anspruch 1, **dadurch gekennzeichnet, dass**
die mehreren Schwingungsmodi einen Biegemodus und einen Ausdehnungs-/Zusammenziehmodus enthalten.

10. Ultraschallbild-Erzeugungsvorrichtung nach Anspruch 1, **dadurch gekennzeichnet, dass**
der Härteberechnungsabschnitt (155) konfiguriert ist, die Härte unter Verwendung des folgenden Ausdrucks zu berechnen:

$$E = \rho Cn L^2 fn^2,$$

wobei

im Ausdruck E die Härte repräsentiert, $\rho$ eine Dichte des Organs repräsentiert, Cn eine Konstante in einem Schwingungsmodus n repräsentiert, L die Größeninformationen repräsentiert und fn eine Eigenfrequenz im Schwingungsmodus n repräsentiert.

11. Ultraschallbild-Erzeugungsvorrichtung nach Anspruch 1, **dadurch gekennzeichnet, dass** das Echosignal, das durch den Geschwindigkeitsberechnungsabschnitt (153) verwendet wird, ein Echosignal ist, das in einer Situation gemessen wurde, in der ein Schwingungserreger konfiguriert ist, eine Schwingung des Organs anzuregen.

12. Ultraschallbild-Erzeugungsvorrichtung nach Anspruch 11, **dadurch gekennzeichnet, dass** die Ultraschallsonde (2) außerdem konfiguriert ist, als der Schwingungserreger zu dienen.

13. Ultraschallbild-Erzeugungsvorrichtung nach Anspruch 1, **dadurch gekennzeichnet, dass** die Ultraschallbild-Erzeugungsvorrichtung ferner einen Anzeigebilderzeugungsabschnitt (157) umfasst, der konfiguriert ist, zu bewirken, dass eine Anzeigevorrichtung ein Ergebnis anzeigt, das durch den Härteberechnungsabschnitt (155) berechnet wurde.

14. Verfahren zum Berechnen der Härte eines Organs unter Verwendung eines Ultraschallempfangssignals, das vom Organ (30) empfangen wird, indem ein Ultraschallsignal zum Organ (30), das in Schwingungen versetzt wird, gesendet wird, wobei das Verfahren **gekennzeichnet ist durch** Berechnen von Geschwindigkeiten von mindestens zwei Orten auf dem Organ (30) unter Verwendung des Ultraschallempfangssignals, Analysieren der Geschwindigkeiten der zwei Orte, um mehrere Schwingungsmodi des Organs (30) und eine Schwingungsfrequenz in jedem der Schwingungsmodi zu identifizieren, und Berechnen einer Härte des Organs unter Verwendung der identifizierten Schwingungsmodi und Schwingungsfrequenzen, von Informationen über eine Größe des Organs (30) und einer Schwingungskonstante, die im Voraus für jeden der mehreren Schwingungsmodi bestimmt wurde, wobei das Organ eine Niere ist und die zwei Orte sich auf einer Seite, von einem Mittelpunkt einer Längsausdehnung der Niere zu einem ihrer Enden verschoben und bei zwei verschiedenen Ultraschalltiefen befinden.

**Revendications**

1. Appareil d'imagerie par ultrasons comprenant :

une section de mesure par ultrasons (10) qui inclut une sonde à ultrasons (2) et qui est configurée pour mesurer un signal écho depuis une région contenant un organe désiré (30) ; et une section de traitement de signal (15) configurée pour effectuer des calculs en utilisant le signal écho, l'appareil d'imagerie par ultrasons étant **caractérisé en ce que** la section de traitement de signal (15) inclut une section de calcul de vitesse (153) configurée pour calculer les vitesses d'au moins deux emplacements sur l'organe (30) en utilisant le signal écho, une section d'identification de mode de vibration (154) configuré pour analyser les vitesses des deux emplacements afin d'identifier une pluralité de modes de vibration de l'organe (30) et une fréquence de vibration dans chacun des modes de vibration, et une section de calcul de dureté (155) configurée pour calculer les duretés de l'organe (30) en utilisant les modes de vibration et les fréquences de vibration identifié(e)s par la section d'identification de mode de vibration, une constante déterminée à l'avance en relation à l'organe, et une information sur une taille de l'organe (30), dans lequel l'organe (30) est un rein, et les deux emplacements sont situés sur un côté décalé depuis un centre d'une longueur longitudinale du rein vers une extrémité de celui-ci et à des profondeurs différentes vis-à-vis des ultrasons.

2. Appareil d'imagerie par ultrasons selon la revendication 1, **caractérisé en ce que** la section de traitement de signal (15) inclut en outre une section de mesure de taille configurée pour calculer les informations concernant une taille de l'organe sur la base d'une image morphologique de l'organe (30).

3. Appareil d'imagerie par ultrasons selon la revendication 2, **caractérisé en ce que** l'image morphologique de l'organe est une image par ultrasons formée à partir du signal écho mesuré par la section de mesure par ultrasons (10).

4. Appareil d'imagerie par ultrasons selon la revendication 1, **caractérisé en ce que** la constante est une valeur calculée en effectuant une simulation qui utilise une expression ci-après et qui accepte une entrée de dureté imaginaire à titre de dureté de l'organe (30) :

$$Cn = E'/\rho L^2 fn^2$$

expression dans laquelle E' représente une dureté imaginaire, p représente une densité de l'organe, Cn représente une constante dans un mode de vibration n, n étant un entier supérieur ou égal à 1, ceci s'appliquant également à la valeur n suivante, L représente l'information de taille, et fn représente une fréquence naturelle dans le mode de vibration n.

5. Appareil d'imagerie par ultrasons selon la revendication 1,
   **caractérisé en ce que** l'appareil d'imagerie par ultrasons comprend en outre une section de mémorisation configurée pour mémoriser la constante.

6. Appareil d'imagerie par ultrasons selon la revendication 1,
   **caractérisé en ce que** la section d'identification de mode de vibration est configurée pour identifier les modes de vibration et les fréquences des modes de vibration en utilisant une différence entre un spectre d'une somme des vitesses des deux emplacements et une somme des spectres aux deux emplacements.

7. Appareil d'imagerie par ultrasons selon la revendication 1,
   **caractérisé en ce que** la section d'identification de mode de vibration (154) est configurée pour identifier les modes de vibration et les fréquences dans les modes de vibration en utilisant une différence de phase entre les spectres des vitesses des deux emplacements.

8. Appareil d'imagerie par ultrasons selon la revendication 1,
   **caractérisé en ce que** l'information sur la taille de l'organe (30) inclut un paramètre quelconque parmi une taille de l'organe le long d'un grand axe de celui-ci, une taille de l'organe (30) le long d'un petit axe de celui-ci, un volume de l'organe, et une aire d'une section transversale prédéterminée de l'organe.

9. Appareil d'imagerie par ultrasons selon la revendication 1,
   **caractérisé en ce que** la pluralité de modes de vibration inclut un mode en flexion et un mode en expansion/contraction.

10. Appareil d'imagerie par ultrasons selon la revendication 1,
    **caractérisé en ce que** la section de calcul de dureté est configurée pour calculer la dureté en utilisant l'expression suivante :

$$E = \rho CnL^2 fn^2,$$

expression dans laquelle E représente la dureté, p représente une densité de l'organe, Cn représente une constante dans un mode de vibration n, L représente l'information de taille, et fn représente une fréquence naturelle dans le mode de vibration n.

11. Appareil d'imagerie par ultrasons selon la revendication 1,
    **caractérisé en ce que** le signal écho utilisé par la section de calcul de vitesse (153) est un signal écho mesuré dans une situation dans laquelle un excitateur de vibration est configuré pour exciter la vibration de l'organe.

12. Appareil d'imagerie par ultrasons selon la revendication 11,
    **caractérisé en ce que** la sonde à ultrasons (2) est également configurée pour servir d'excitateur de vibration.

13. Appareil d'imagerie par ultrasons selon la revendication 1,
    **caractérisé en ce que** l'appareil d'imagerie par ultrasons comprend en outre une section de création d'image d'affichage (157) configurée pour amener un dispositif d'affichage à afficher un résultat calculé par la section de calcul de dureté (155).

14. Procédé pour calculer la dureté d'un organe en utilisant un signal de réception ultrasonore reçu depuis l'organe (30) en émettant un signal ultrasonore vers l'organe (30) ainsi amené à vibrer,
    **caractérisé en ce que** le procédé comprend les étapes consistant à :

    calculer les vitesses d'au moins deux emplacements sur l'organe (30) en utilisant le signal de réception ultrasonore,
    analyser les vitesses des deux emplacements pour identifier une pluralité de modes de vibration de l'organe (30) et une fréquence de vibration dans chacun des modes de vibration, et
    calculer la dureté ledit organe en utilisant les modes de vibration identifiés et les fréquences de vibration identifiées, l'information sur une taille de l'organe (30), et une constante de vibration calculée à l'avance pour chacun de la pluralité de modes de vibration,
    dans lequel l'organe est un rein et les deux emplacements sont situés sur un côté décalé depuis un centre d'une longueur longitudinale du rein vers une extrémité de celui-ci et à différentes profondeurs vis-à-vis des ultrasons.

[FIG. 1]

[FIG. 2]

[FIG. 3]

[FIG. 4]

|  |  | RIGHT | LEFT | AVERAGE | DIFFERENCE [%] |
|---|---|---|---|---|---|
| BENDING MODE | $L^2 = a^2$ | 5.3 | 4.9 | 5.1 | 7.5 |
|  | $L^2 = ab$ | 7.4 | 7.4 | 7.4 | -0.2 |
| EXTENSION/ CONTRACTION MODE | $L^2 = a^2$ | 3.2 | 2.8 | 3.0 | 13.9 |
|  | $L^2 = ab$ | 4.5 | 4.2 | 4.3 | 6.2 |

[FIG. 5]

(a) BENDING MODE

(b) EXTENSION/ CONTRACTION MODE

[FIG. 6]

```
                    ┌─────────────┐
                    │    START    │
                    └─────────────┘
                           │
                           ▼
          ┌──────────────────────────────┐
          │     ACQUIRE TOMOGRAPHIC       │ ⟿ S601
          │            IMAGE             │
          └──────────────────────────────┘
                           │
                           ▼
          ┌──────────────────────────────┐
          │         MEASURE SIZE         │ ⟿ S602
          └──────────────────────────────┘
                           │
                           ▼
          ┌──────────────────────────────┐
          │   PERFORM M-MODE IMAGING/    │ ⟿ S603
          │       EXCITE VIBRATION       │
          └──────────────────────────────┘
                           │
                           ▼
          ┌──────────────────────────────┐
          │    IDENTIFY VIBRATION MODE   │ ⟿ S604
          └──────────────────────────────┘
                           │
                           ▼
          ┌──────────────────────────────┐
          │      CALCULATE HARDNESS      │ ⟿ S605
          └──────────────────────────────┘
                           │
                           ▼
          ┌──────────────────────────────┐
          │        DISPLAY RESULT        │ ⟿ S606
          └──────────────────────────────┘
                           │
                           ▼
                    ┌─────────────┐
                    │     END     │
                    └─────────────┘
```

[FIG. 7]

[FIG. 8]

[FIG. 9]

[FIG. 10]

EXTERNAL VIBRATION EXCITING DEVICE

[FIG. 11]

```
           ┌─────────────┐
           │    START    │
           └─────────────┘
                  │
                  ▼
    ┌──────────────────────────┐
    │ CALCULATE SPEED BETWEEN  │ ～～ S1101
    │       TWO POINTS         │
    └──────────────────────────┘
                  │
                  ▼
    ┌──────────────────────────┐
    │  CALCULATE SPECTRUM AT   │
    │     TWO POINTS AND       │ ～～ S1102
    │    SPECTRUM SUM 910      │
    └──────────────────────────┘
                  │
                  ▼
    ┌──────────────────────────┐
    │  CALCULATE SPECTRUM OF   │ ～～ S1103
    │     SPEED SUM 920        │
    └──────────────────────────┘
                  │
                  ▼
    ┌──────────────────────────┐
    │   CALCULATE DIFFERENCE   │
    │ BETWEEN SPECTRUM 910 AND │ ～～ S1104
    │     SPECTRUM 920         │
    └──────────────────────────┘
                  │
                  ▼
    ┌──────────────────────────┐
    │  IDENTIFY VIBRATION MODE │ ～～ S1105
    └──────────────────────────┘
                  │
                  ▼
           ┌─────────────┐
           │     END     │
           └─────────────┘
```

[FIG. 12]

SPEED OF POINT B

SPEED OF POINT A

SPEED

TIME

[FIG. 13]

(a)

(b)

[FIG. 14]

(a) E=50kPa

(b)

BENDING MODE / EXPANSION/CONTRACTION MODE

MAGNITUDE OF VIBRATION

FREQUENCY

(c)

BENDING MODE / EXPANSION/CONTRACTION MODE

[FIG. 15]

(a) TWICE MEASUREMENT

(b) DUAL DOPPLER

(c) SPARSE IMAGING

(d) DEFOCUS IMAGING

[FIG. 16]

```
         ┌───────────┐
         │   START   │
         └───────────┘
               │
               ▼
  ┌─────────────────────────┐
  │   CALCULATE SPECTRA     │          S1601
  │     AT TWO POINTS       │
  └─────────────────────────┘
               │
               ▼
  ┌─────────────────────────┐
  │       CALCULATE         │          S1602
  │    PHASE DIFFERENCE     │
  │    BETWEEN SPECTRA      │
  └─────────────────────────┘
               │
               ▼
  ┌─────────────────────────┐
  │        IDENTIFY         │          S1603
  │   FIRST VIBRATION MODE  │
  └─────────────────────────┘
               │
               ▼
  ┌─────────────────────────┐
  │        IDENTIFY         │          S1604
  │  SECOND VIBRATION MODE  │
  └─────────────────────────┘
               │
               ▼
         ┌───────────┐
         │    END    │
         └───────────┘
```

[FIG. 17]

(a)

SPEED OF POINT B

SPEED

TIME

SPEED OF POINT A

(b)

**EXTENSION/
CONTRACTION MODE**

PHASE DIFFERENCE

PHASE DIFFERENCE
BETWEEN SPECTRA

FREQUENCY

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011102221 A

- EP 2537469 A1

**Non-patent literature cited in the description**

- **HONDA, H et al.** *Am J Physiol Heart Circ Physiol,* 1994, vol. 266, H881-H890